# EUROPEAN PATENT APPLICATION

(11) **EP 4 266 033 A1**
(43) Date of publication of application: **25.10.2023**
(21) Application number: 21907161.0
(22) Date of filing: 17.12.2021
(51) Int. Cl.: G01N 27/02, G01N 27/30, G01N 33/569, C12Q 1/02

(54) **BIOSENSOR USING PET SUBSTRATE, FABRICATION METHOD THEREFOR, AND METHOD FOR INSPECTING SENSITIVITY OF MICROORGANISM TO ANTIBIOTIC BY USING SAME**

(30) Priority: 18.12.2020 KR 20200178756
(71) Applicant: Proteometech Inc., Seoul 07528 (KR)
(72) Inventor: LIM, Kook Jin, Seoul 07324 (KR); JOO, Han Seung, Seoul 08003 (KR); OH, Je Seung, Gwangju-si, Gyeonggi-do 12791 (KR); LIM, Chang Su, Asan-si, Chungcheongnam-do 31486 (KR)
(74) Representative: Maiwald GmbH
(86) International application number: PCT/KR2021/019292
(87) International publication number: WO 2022/131857

(57) **Abstract**

The present invention relates to a biosensor for inspecting the susceptibility of a microorganism to an antibiotic, a fabrication method therefor, and a method for inspecting the susceptibility of a microorganism to an antibiotic by using same, wherein the biosensor comprises: a substrate including polyethylene terephthalate; and a sensor part formed on one surface or both surfaces of the substrate and comprising an electrode inclusive of a first electrode and a second electrode which interdigitate with each other.

## Description

### [Technical Field]

The present invention relates to a biosensor using a PET substrate, a method for manufacturing the same and a method for inspecting the susceptibility of a microorganism to an antibiotic using the same.

### [Background Art]

Among various diseases occurring in the human body, there are diseases caused by infection with microorganisms including urinary tract infection and sepsis. In order to treat diseases caused by microbial infection, antibiotics that inhibit the growth of microorganisms are required, and these antibiotics inhibit the growth of microorganisms or kill microorganisms. However, more than 100 types of antibiotics have been developed until recently, and their effects differ depending on the type of microorganism or whether the microorganism is resistant to antibiotics. Therefore, it is necessary to select the most effective antibiotic for the corresponding microorganism for infection patients, and the diagnostic test method therefor is called the antibiotic susceptibility test.

Currently, the most commonly used antibiotic susceptibility test method is the broth dilution method, and in this method, after culturing bacteria for more than one day in antibiotic-treated culture medium under various conditions, antibiotic resistance and susceptibility are determined by identifying bacteria and measuring the turbidity of the culture medium, and it has a disadvantage that this process requires a lot of time and labor. Therefore, the situation is that it is necessary to develop a method for solving the problems of the existing antibiotic susceptibility test method by enabling a real-time test and reducing the time and labor required for the test.

### [Related Art Documents]

### [Patent Documents]

Korean Registered Patent No. 10-1776698 (September 4, 2017)

### [Disclosure]

### [Technical Problem]

The present invention is directed to providing a biosensor for inspecting the susceptibility of a microorganism to an antibiotic and the like, including a substrate which includes polyethylene terephthalate; and an electrode layer which is formed on one surface or both surfaces of the substrate and includes a first electrode and a second electrode which interdigitate with each other.

However, the technical problems to be achieved by the present invention is not limited to the above-mentioned problems, and other problems that are not mentioned will be clearly understood by those skilled in the art from the following description.

### [Technical Solution]

The present invention provides a biosensor for inspecting the susceptibility of a microorganism to an antibiotic, including a substrate which includes polyethylene terephthalate; and an electrode layer which is formed on one surface or both surfaces of the substrate and includes a first electrode and a second electrode which interdigitate with each other.

The electrode may be made of silver or gold.

The electrode may be formed through screen printing, PCB or photolithography.

The biosensor may be for inspecting the susceptibility of a microorganism to an antibiotic by measuring in real time a change in electrical properties occurring as the microorganism grows according to the treatment with the antibiotic.

In an embodiment of the present invention, provided is a method for preparing the biosensor, including the steps of preparing a substrate which includes polyethylene terephthalate; and forming an electrode which includes a first electrode and a second electrode which interdigitate with each other on the substrate.

In another embodiment of the present invention, providing a method for inspecting the susceptibility of a microorganism to an antibiotic, including the steps of treating a sample containing a microorganism on the biosensor; treating the sample-treated biosensor with an antibiotic; and measuring a change in electrical properties, after treatment with the antibiotic.

### [Advantageous Effects]

Since the biosensor according to the present invention is characterized by including a substrate which includes polyethylene terephthalate; and an electrode which is formed on one surface or both surfaces of the substrate and includes a first electrode and a second electrode which interdigitate with each other, it has the advantages that the time required to detect the growth change of a microorganism is short and the discrimination is excellent, compared to the case of using other material substrates. Moreover, not only can the rapid inspection of the susceptibility of a microorganism to an antibiotic be possible, but also the minimum antibiotic concentration that is capable of limiting resistance can be rapidly identified.

Therefore, when the biosensor according to the present invention is used, the time required for the inspection of the susceptibility of a microorganism to an antibiotic can be dramatically reduced to less than 4 hours due to the remarkably excellent susceptibility, and thus, it can be very effectively applied in treating microbial infections requiring rapid judgment and treatment.

### [Description of Drawings]

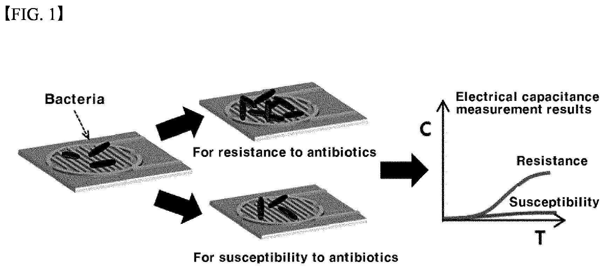

FIG. 1 is a diagram schematically showing the method for inspecting the susceptibility of a microorganism to an antibiotic by using the biosensor using a PET substrate according to an embodiment of the present invention.
FIGS. 2a and 2b are diagrams schematically showing the biosensor using a PET substrate and an Ag electrode according to an embodiment of the present invention.
FIGS. 3a and 3b are diagrams schematically showing the biosensor using a PET substrate and an Au electrode according to an embodiment of the present invention.
FIG. 4a is a graph for measuring the capacitance change of a microorganism when the biosensor using a PET substrate and an Ag electrode according to an embodiment of the present invention is used, and FIG. 4b is a graph for measuring the results of inspecting the susceptibility of a microorganism to an antibiotic when the biosensor using a PET substrate and an Ag electrode according to an embodiment of the present invention is used.
FIGS. 5a and b are graphs for measuring the capacitance change of a microorganism when the biosensor using a PET substrate and an Au electrode according to an embodiment of the present invention is used, and FIG. 5c is a graph for measuring the results of inspecting the susceptibility of a microorganism to an antibiotic when the biosensor using a PET substrate and an Au electrode according to an embodiment of the present invention is used.

### [Modes of the Invention]

The inventors of the present invention studied the biosensor for inspecting the susceptibility of a microorganism to an antibiotic by measuring changes in electrical properties that occur during the growth of microorganisms in real time according to the antibiotic treatment, and when polyethylene terephthalate was applied as an optimal material for the substrate, it was confirmed that the susceptibility was remarkably excellent, thereby completing the present invention.

Hereinafter referred to as, the present invention will be described in detail.

However, since the present invention can have various changes and have various forms, the specific examples and descriptions described below are only for helping the understanding of the present invention, and are not intended to limit the present invention to specific disclosed forms. It should be understood that the scope of the present invention includes all modifications, equivalents and substitutes included in the spirit and scope of the present invention.

In the present specification, the term "susceptibility to an antibiotic" is also referred to as antibiotic susceptibility, and the microorganism is affected by the inhibition of growth, the disruption of division, cell death and the like by a specific antibiotic, and according to the antibiotic susceptibility test method of the conventional disk diffusion method, a circular clear zone having a certain diameter is formed around the antibiotic disk, and when the size of this diameter exceeds a certain level, it is said to have antibiotic susceptibility. Antibiotic susceptibility test results can be divided into, for example, susceptibility, intermediate resistance and resistance (tolerance). In order to treat infections caused by microorganisms, antibiotics that are susceptible to the microorganisms must be used, and in the case of microorganisms that are read as being susceptible, it means that they can be treated by the prescription of antibiotics at a dose recommended for the species and infection site, and in the case of microorganisms that are read as exhibiting the intermediate resistance, the minimum growth inhibitory concentration of the antibiotic for the target microorganism is similar to the maximum concentration of the drug that can be prescribed, which means that the therapeutic effect may be reduced. Meanwhile, in the case of microorganisms that are read as exhibiting the resistance (tolerance), it indicates that they are not treated at the maximum concentration of the drug that can be prescribed.

Biosensor for inspecting susceptibility of microorganism to antibiotic and preparation method thereof

The present invention provides a biosensor for inspecting the susceptibility of a microorganism to an antibiotic, including a substrate which includes polyethylene terephthalate; and an electrode layer which is formed on one surface or both surfaces of the substrate and includes a first electrode and a second electrode which interdigitate with each other.

In addition, the present invention provides a method for preparing the biosensor, including the steps of preparing a substrate which includes polyethylene terephthalate; and forming an electrode layer which includes a first electrode and a second electrode which interdigitate with each other on the substrate.

The biosensor according to the present invention is for inspecting the susceptibility of a microorganism to an antibiotic, and specifically, according to antibiotic treatment, changes in electrical properties that occur while the microorganisms grow are measured in real time to test the susceptibility of a microorganism to an antibiotic. In this case, the electrical properties may be capacitance, impedance, resistance, reactance or the like. Therefore, when measuring the changes in electrical properties, there is an advantage in that susceptibility can be increased even with a lower concentration and a small amount of sample, compared to when measuring the changes in optical properties.

First of all, the biosensor according to the present invention includes a substrate which includes polyethylene terephthalate. The polyethylene terephthalate is a non-conductive polymer, and has advantages in that the time required for detecting a change in the growth of microorganisms is short and the discrimination is excellent, compared to the case of using a substrate made of other materials such as epoxy, polyimide, glass or the like. Moreover, it is possible not only to rapidly inspect the susceptibility of a microorganism to an antibiotic, but also to quickly identify the minimum concentration of an antibiotic that can limit resistance.

Next, the biosensor according to the present invention includes an electrode layer which includes a first electrode and a second electrode that interdigitate with each other, wherein the electrode layer is formed on one or both surfaces of the substrate. The electrode layer may have a structure in which rods that are arranged in a row form one pole, and two electrodes (a first electrode and a second electrode) face each other while being engaged with each other. The two electrodes act like a classical impedance measuring electrode. The distance between the first electrode and the second electrode may be 0.1 µm to 1,000 µm, 1 µm to 500 µm, or 10 µm to 300 µm. By adjusting the distance between the first electrode and the second electrode within the above range, it is possible to precisely measure even micro-level biomolecules. In addition, the height of the first electrode and the second electrode may range from 50 nm to 5,000 nm, and the width of the first electrode and the second electrode may be 0.1 µm to 1,000 µm, 1 µm to 500 µm, or 10 µm to 300 µm. Specifically, the electrode may be made of silver or gold.

The electrode may be formed through screen printing, PCB or photolithography. Specifically, when the electrode is made of silver, the silver electrode may be formed through the screen printing method. In addition, when the electrode is made of a gold material, the gold electrode may be formed through the PCB method. In the case of the screen printing or PCB method, there is an advantage in that the manufacturing cost is 1/100 lower than that of the photolithography method.

The biosensor according to the present invention may further include an accommodating part. The accommodating part may accommodate an electrode layer, an antibiotic and a microorganism therein. In addition, the accommodating part may be formed in a direction perpendicular to the substrate. Specifically, when measuring the susceptibility of a microorganism to an antibiotic by using the biosensor, microorganisms or antibiotics may be treated inside the accommodating part. The accommodating part may have an open top.

The accommodating part may be made of one or more materials selected from the group consisting of glass, polypropylene (PP), polyethylene terephthalate (PET) and polycarbonate (PC). As an example that is commercially used, it may be a well that is made of a plastic material. The capacity of the accommodating part may be from 10 µL to 1 mL. The microorganism may be treated together with a culture medium, and in this sense, the accommodating part may also include a culture medium of the microorganism therein.

The culture medium is also referred to as a medium or a culture solution, and it refers to a liquid or solid material used for the growth and preservation of microorganisms, and the specific composition thereof may be different depending on the type of microorganism. Liquid form is preferred, and it may be or include blood.

The microorganism may be a bacterium, and the bacterium is preferably a gram-positive bacterium, a gram-negative bacterium and an antibiotic-resistant strain thereof, but the present invention is not limited thereto. Specifically, the gram-positive bacterium may be at least one selected from the group consisting of *Bacillus subtilis, Staphylococcus aureus, Enterococcus faecalis* and *Staphylococcus epidermidis,* and the gram-negative bacterium may be at least one selected from the group consisting of *Escherichia coli, Pseudomonas aeruginosa, Acinetobacter baumannii* and *Salmonella typhimurium.*

The antibiotic is not specifically limited to the type thereof, and any antibiotic that can measure the susceptibility with the biosensor according to the present invention is included. Specifically, the antibiotic may be selected from the group consisting of Ampicillin, Tetracycline, Gentamicin, Erythromycin, Vancomycin, Linezolid, Methicillin, Oxacillin, Cefotaxime, Rifampicin, Amikacin, Amikacin, Kanamycin, Tobramycin, Neomycin, Ertapenem, Doripenem, Imipenem/Cilastatin, Meropenem, Ceftazidime, Cefepime, Ceftaroline, Ceftobiprole, Aztreonam, Piperacillin, Polymyxin B, Colistin, Ciprofloxacin, Levofloxacin, Moxifloxacin, Gatifloxacin, Tigecycline, a combination thereof and a derivative thereof, and it is preferably Ampicillin or Tetracycline, but the present invention is not limited thereto.

The biosensor measuring device according to the present invention may further include a wireless transmission unit for transmitting the measurement result of a change in electrical properties, and this transmission method may be performed by the conventionally used method including all of wired data transmission using a data cable such as USB or wireless method using the Bluetooth method.

The biosensor according to the present invention can be connected to a measuring instrument which is capable of measuring electrical properties to monitor changes in the concentration of a microorganism in real time, and in this case, an AC voltage of 1 mV to 1 V having a frequency of 10 Hz to 1 MHz may be supplied to the biosensor.

### Method for inspecting susceptibility of microorganism to antibiotic

The present invention provides a method for inspecting the susceptibility of a microorganism to an antibiotic, including the steps of treating a sample containing a microorganism on the biosensor; treating the sample-treated biosensor with an antibiotic; and measuring a change in electrical properties, after treatment with the antibiotic.

First of all, the method for inspecting the susceptibility of a microorganism to an antibiotic according to the present invention includes the step of treating a sample containing the microorganism on the biosensor.

Since the biosensor has been described above, the redundant description thereof will be omitted.

In addition, the sample contains a microorganism, and since the microorganism has been described above, the redundant description will be omitted. The sample may be in the form of a composition, and may be collected from any one of liquid, soil, air, food, waste, the intestines of animals and plants and tissues derived from animals and plants, blood, urine, tears, saliva, or sweat, and the animals and plants include the human body. The sample may also be a microorganism that is extracted from a sample from human blood and cultured in a blood culture medium or a separate culture medium and amplified. For the sample, a sample in which the species is not identified by a mass spectrometer or other identification kit may be used.

Next, the method for inspecting the susceptibility of a microorganism to an antibiotic according to the present invention includes the step of treating the sample-treated biosensor with an antibiotic.

Since the antibiotic has been described above, the redundant description thereof will be omitted.

Next, the method for inspecting the susceptibility of a microorganism to an antibiotic according to the present invention includes the step of measuring a change in electrical properties, after treatment with the antibiotic.

Specifically, by measuring the changed electrical properties before and after antibiotic treatment, the antibiotic susceptibility can quickly check the antibiotic resistance and the survival of microorganisms according to the antibiotic resistance according to the change value in real time, and resistance can be determined more precisely through changes in electrical properties such as capacitance, impedance, resistance, reactance and the like. For example, the minimum inhibitory concentration (MIC) value of an antibiotic according to the antibiotic concentration may also be measured, and since the optimal antibiotic can be selected according to resistance, it is possible to prevent the misuse and abuse of antibiotics, and there is an advantage in that side effects can be reduced accordingly.

As discussed above, since the biosensor according to the present invention is characterized by including a substrate which includes polyethylene terephthalate; and an electrode layer which is formed on one surface or both surfaces of the substrate and includes a first electrode and a second electrode which interdigitate with each other, it has advantages in that the time required to detect the growth change of microorganisms is short and it has excellent discrimination, compared to the case of using other material substrates. Moreover, not only can rapid antimicrobial susceptibility testing of microorganisms be possible, but also the minimum antibiotic concentration that can limit resistance can be rapidly identified.

Therefore, when the biosensor according to the present invention is used, the time required for the antibiotic susceptibility test of microorganisms can be dramatically reduced to less than 4 hours due to the remarkably excellent susceptibility, and thus, it can be very effectively applied in treating microbial infections requiring rapid judgment and treatment.

Hereinafter, preferred examples are presented to help the understanding of the present invention. However, the following examples are only provided for easier understanding of the present invention, and the contents of the present invention are not limited by the following examples.

### <Example>

### Preparation Example 1: Preparation of the experiment

The strain used in the experiment was *Escherichia coli* (ATCC25922), and a standard strain was purchased from ATCC of the United States and used. For the culture of the strain, the Mueller-Hinton media (MH media), which is a culture medium recommended by the US standard CLSI and European standard EUCAST, was purchased from DIFCO and used according to the recommended preparation method in the product manual. After culturing the strain in the MH media for 24 hours, the strain culture medium was adjusted to McFarland OD 0.5, and then diluted 1/100 with the culture medium (strain concentration: 5 × 10⁵ to 10⁶ cfu/mL) and treated on the sensor.

Meanwhile, the antibiotics used in the experiment were ampicillin and tetracycline, which were purchased from Sigma, and depending on the nature of each antibiotic, it may be used by dissolving in deionized and autoclaved water, 100% DMSO or 100% ethanol (different solvents may be variously used for each antibiotic. For example, Tetracycline can be used by dissolving in 100% ethanol and diluting with deionized water that has been autoclaved) to be made at a concentration of 8 mg/mL, and after filtering through a 22 µm filter, it was diluted with autoclaved and deionized water to a concentration of 0.5 to 8 µg/mL and treated on the sensor.

### Example 1: Preparation of biosensor (PET substrate-Ag electrode) for evaluating susceptibility of microorganism to antibiotic

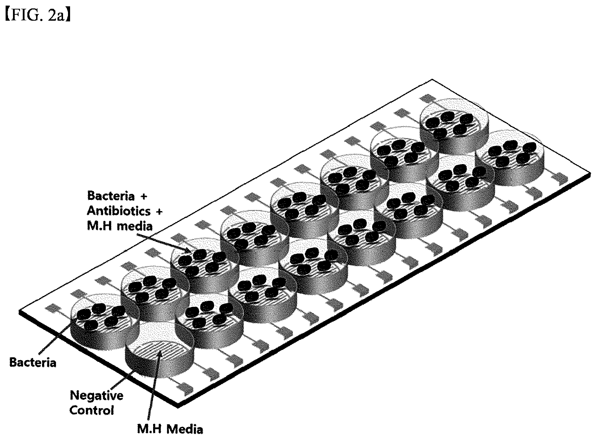

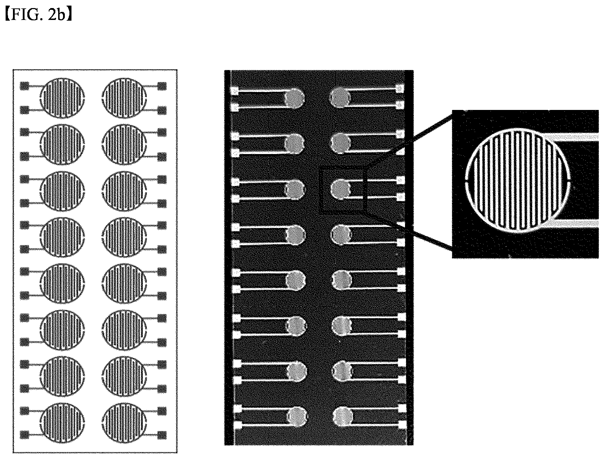

In order to fabricate a biosensor for evaluating the susceptibility of a microorganism to an antibiotic, micro-silver ink (Ebase SC-PE09F4 datasheet) was printed on a non-conductive polymer polyethylene terephthalate (PET) (SKC product, SW94M) material substrate (thickness: 250 µm) by the screen printing method (screen printing process) to fabricate a sensor in which an interdigitated Ag electrode (thickness: ~150 µm) was formed (refer to FIGS. 2(a) and 2(b)). In this case, for 16-channel and 64-channel interdigitated sensors, the electrode height (~150 µm), width (100 µm), and electrode spacing of 100 µm were set to the sensor size of 4.5 mm in diameter. Thereafter, the accommodating part that was manufactured by the injection method by using a polypropylene material on the Ag electrode was attached by using a waterproof double-sided tape or a heat sealing tape, and it was finally sterilized by using O₃. In addition, the Ag electrode and the pad for measuring an electrical signal were allowed to conduct electricity through a via hole.

### Comparative Example 1: Preparation of biosensor (epoxy substrate-Ag electrode) for evaluating susceptibility of microorganism to antibiotic

A sensor was fabricated in the same manner as in Example 1, except that an epoxy substrate was used instead of the PET substrate.

### Example 2: Preparation of biosensor (PET substrate-Au electrode) for evaluating susceptibility of microorganism to antibiotic

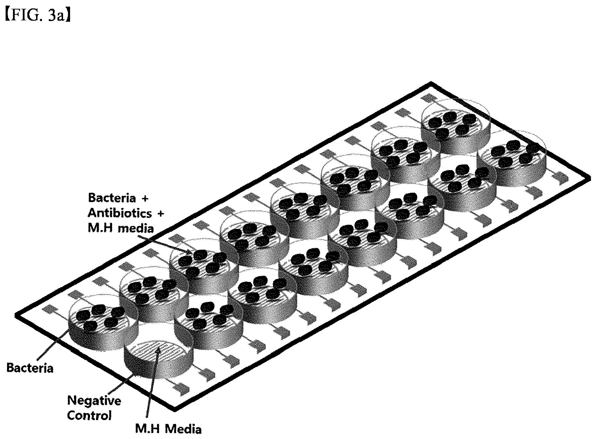

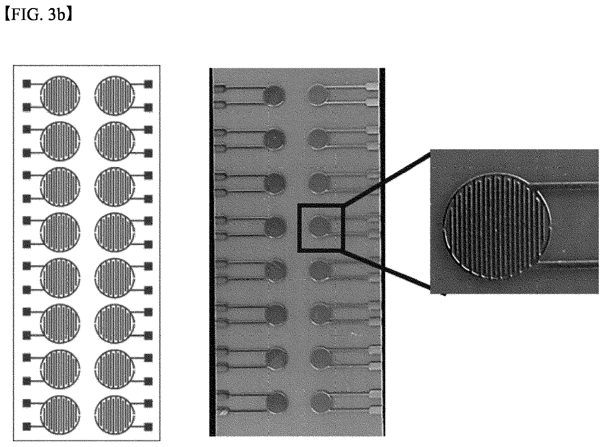

In order to fabricate a biosensor for evaluating the susceptibility of a microorganism to an antibiotic, double-sided printing was performed by the PCB manufacturing process method through non-electrolysis and electroplating on a non-conductive polymer polyethylene terephthalate (PET) (SKC product, SW94M) material substrate (thickness: 250 µm) by the screen printing method (screen printing process) to fabricate a sensor having an Au electrode (thickness: Total: 80.1 µm, Cu 50 µm, Ni: 30 µm, Au 0.1 µm) (refer to FIGS. 3(a) and (b)). In this case, for 16-channel and 64-channel interdigitated Au sensors, the electrode height (~80 µm), width (100 µm), and electrode spacing of 100 µm were set to the sensor size of 2.5 mm in diameter. Thereafter, the accommodating part that was manufactured by the injection method by using a polypropylene material on the Au electrode was attached by using a waterproof double-sided tape or a heat sealing tape, and it was finally sterilized by using O₃. In addition, the Au electrode and the pad for measuring an electrical signal were allowed to conduct electricity through a via hole.

### Comparative Example 2: Preparation of biosensor (epoxy substrate-Au electrode) for evaluating susceptibility of microorganism to antibiotic

A sensor was fabricated in the same manner as in Example 2, except that an epoxy substrate was used instead of the PET substrate.

### Comparative Example 3: Preparation of biosensor (polyimide substrate-Au electrode) for evaluating susceptibility of microorganism to antibiotic

A sensor was fabricated in the same manner as in Example 2, except that a polyimide substrate was used instead of the PET substrate.

### Comparative Example 4: Preparation of biosensor (glass substrate-Au electrode) for evaluating susceptibility of microorganism to antibiotic

A sensor was fabricated in the same manner as in Example 2, except that a glass substrate was used instead of the PET substrate and the photolithography method was applied instead of the PCB method.

### Experimental Example 1: Measurement of changes in capacitance of microorganism according to sensor

By using the sensors fabricated in Examples 1 to 2 and Comparative Examples 1 to 4, the ability to measure changes in capacitance of microorganisms was confirmed. Specifically, *E. coli* (ATCC25922) was treated at 10⁵ to 10⁷ cells/mL for each of the sensors fabricated in Examples 1 to 2 and Comparative Examples 1 to 3, and then cultured at 37°C in the MH media, and changes in capacitance according to the growth of *E*. *coli* (ATCC25922) were measured. Meanwhile, with respect to the sensor fabricated in Example 4, after E. *coli* (*E. coli* U556 or *E. coli* U433) or *Staphylococcus aureus* (*S. aureus* T82 or *S*. *aureus* P101) were treated at 1 × 10⁵ cells/mL, it was cultured at 37°C, and changes in capacitance according to the growth of *E*. *coli* (*E. coli* U556 or *E*. *coli* U433) or *S*. *aureus* (*S. aureus* T82 or *S*. *aureus* P101) were measured.

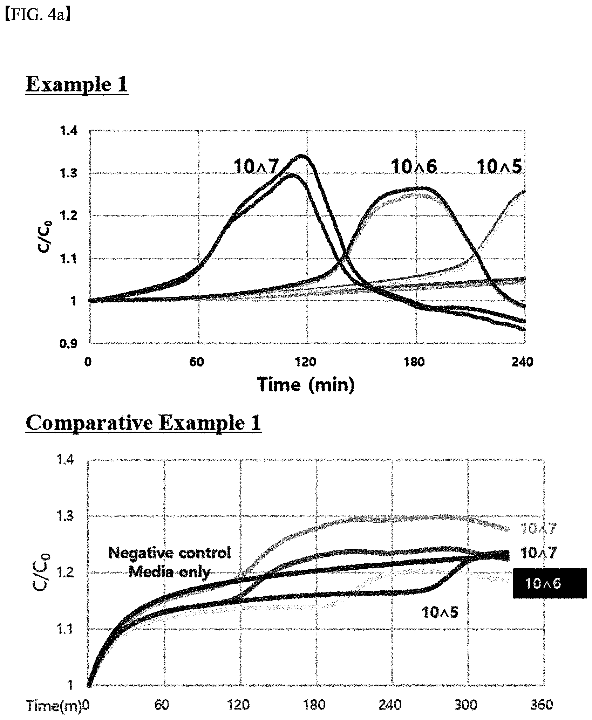

As shown in FIG. 4a, the sensor fabricated in Example 1 applied a PET substrate-Ag electrode, and a large capacitance change was measured within a short period of time, and as a result, it was confirmed that the time required to detect the change in the growth of microorganisms was short and the discrimination was excellent. Meanwhile, the sensor fabricated in Comparative Example 1 applied an epoxy substrate-Ag electrode, and it was confirmed that the capacitance change was only insignificant.

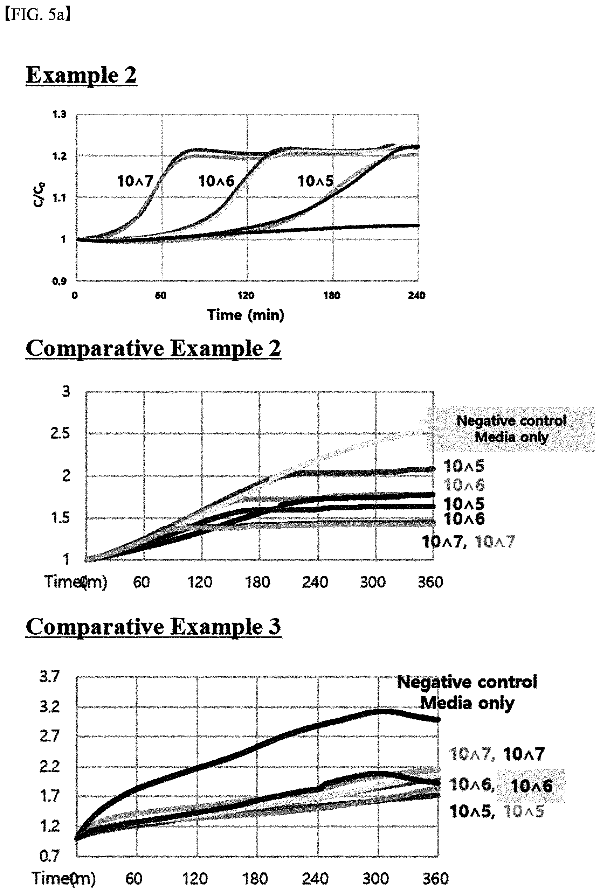

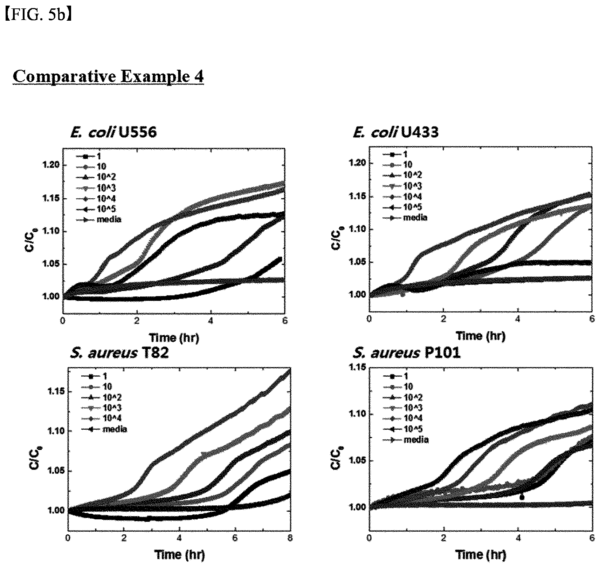

As shown in FIGS. 5a and b, the sensor fabricated in Example 2 applied a PET substrate-Au electrode, and likewise, a large capacitance change was measured in a period of short time, and it was confirmed that the time required to detect the growth change of microorganisms is short and the discrimination power is excellent. Meanwhile, the sensors fabricated in Comparative Examples 2 to 3 applied an epoxy or polyimide substrate-Ag electrode, and it was confirmed that the capacitance change was only insignificant in both. In addition, the sensor fabricated in Comparative Example 4 applied the conventional glass substrate-Au electrode, and in this case, it was confirmed that the capacitance change was only insignificant.

### Experimental Example 2: Susceptibility test of microorganism to antibiotic according to sensor

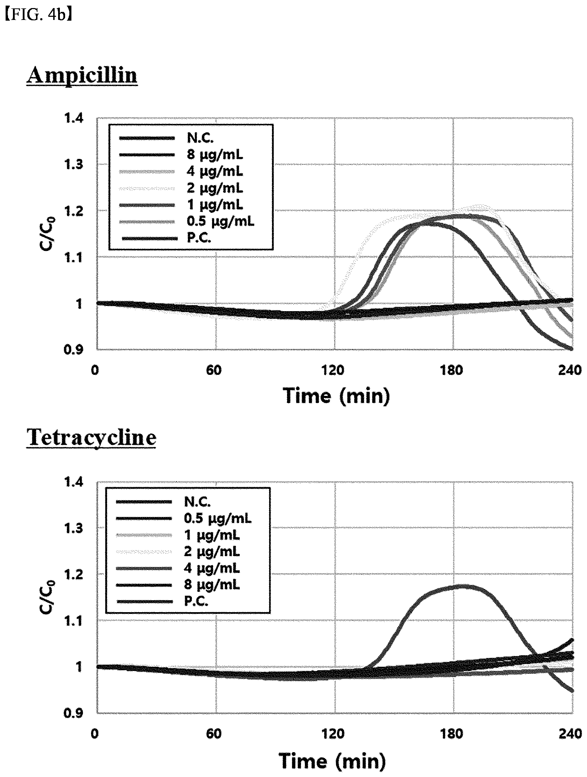

The susceptibility of microorganisms to antibiotics was measured by using the sensors fabricated in Examples 1 and 2. Specifically, for each of the sensors fabricated in Examples 1 and 2, ampicillin at a concentration of 0.5 to 8 µg/mL and tetracycline at a concentration of 0.5 to 8 µg/mL were prepared, and immediately after treating the same on the sensor surface, the prepared *E. coli* (ATCC25922) was treated at 10⁵ cells/mL, and the susceptibility of microorganisms to antibiotics was measured based on the capacitance change results that were monitored in real time. As shown in FIG. 4b, the sensor fabricated in Example 1 applied a PET substrate-Ag electrode, and a significantly increased capacitance change was confirmed after treatment with ampicillin, whereas no change in capacitance was confirmed after treatment with tetracycline. As a result, it is confirmed that *E. coli* (ATCC25922) has tolerance (resistance) to ampicillin and susceptibility to tetracycline.

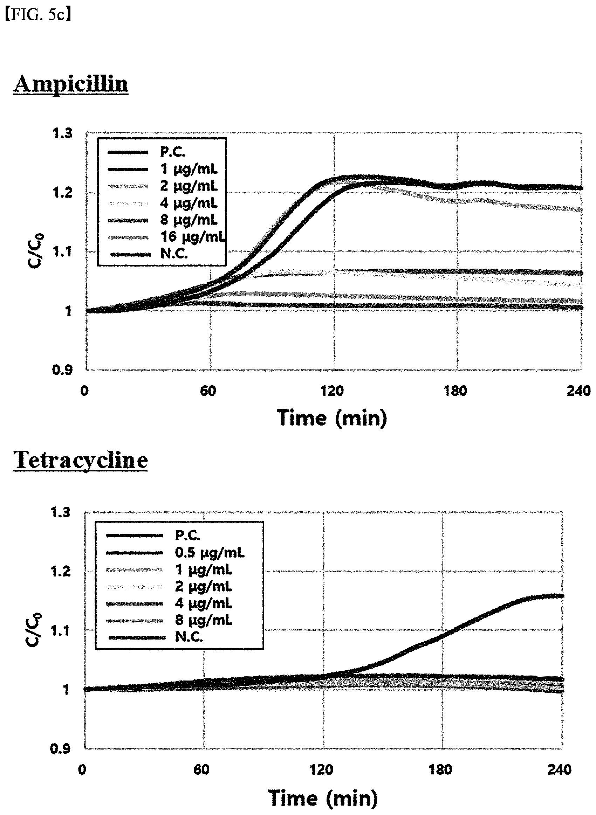

As shown in FIG. 5c, the sensor fabricated in Example 2 applied a PET substrate-Au electrode, and similarly, it was confirmed that a capacitance change significantly increased after treatment with ampicillin, whereas no change in capacitance was confirmed after treatment with tetracycline. As a result, it was confirmed that *E. coli* (ATCC25922) has resistance to ampicillin and susceptible to tetracycline.

That is, when the sensors fabricated in Examples 1 and 2 were used, in the case of treating microorganisms that are resistant to ampicillin followed by treating with ampicillin, capacitance changes that caused a large change were confirmed within 1 to 2 hours. Therefore, when the sensors fabricated in Examples 1 and 2 are used, it is possible to rapidly inspect the susceptibility of a microorganism to an antibiotic. Further, in the case of a multi-channel interdigitated sensor, it can measure several antibiotics at the same time, and thus, it is possible to rapidly provide information on highly sensitive antibiotics. In addition, it has the advantage of being able to quickly identify the minimum antibiotic concentration that can limit resistance.

The above description of the present invention is for illustration, and those of ordinary skill in the art to which the present invention pertains will understand that it can be easily modified into other specific forms without changing the technical spirit or essential features of the present invention. Therefore, it should be understood that the exemplary embodiments described above are illustrative in all respects and not restrictive.

## Claims

1. A biosensor for inspecting the susceptibility of a microorganism to an antibiotic, comprising:
a substrate which comprises polyethylene terephthalate; and
an electrode layer which is formed on one surface or both surfaces of the substrate and comprises a first electrode and a second electrode which interdigitate with each other.

2. The biosensor of claim 1, wherein the electrode is made of silver or gold.

3. The biosensor of claim 2, wherein the electrode is formed through screen printing, PCB or photolithography.

4. The biosensor of claim 1, wherein the biosensor is for inspecting the susceptibility of a microorganism to an antibiotic by measuring in real time a change in electrical properties occurring as the microorganism grows according to the treatment with the antibiotic.

5. A method for preparing the biosensor according to claim 1, comprising the steps of:
preparing a substrate which comprises polyethylene terephthalate; and
forming an electrode which comprises a first electrode and a second electrode which interdigitate with each other on the substrate.

6. A method for inspecting the susceptibility of a microorganism to an antibiotic, comprising the steps of:
treating a sample containing a microorganism on the biosensor according to claim 1;
treating the sample-treated biosensor with an antibiotic; and
measuring a change in electrical, after treatment with the antibiotic.
